# EUROPEAN PATENT APPLICATION

(11) **EP 4 464 353 A1**
(43) Date of publication of application: **20.11.2024**
(21) Application number: 23173331.2
(22) Date of filing: 15.05.2023
(51) Int. Cl.: A61M 11/00, A61M 15/00, A61M 16/00

(54) **INHALATION THERAPY DEVICE**

(71) Applicant: PARI Pharma GmbH, 82319 Starnberg (DE)
(72) Inventor: WEHNER, Daniel, 81243 München (DE); HOFFMANN, Tobias, 86938 Schondorf am Ammersee (DE); HEINE, Benjamin, 80687 München (DE); LACHMAYR, Stefanie, 86415 Mering (DE)
(74) Representative: Hoffmann Eitle

(57) **Abstract**

The present disclosure refers to an inhalation therapy device (1). The inhalation therapy device comprises a housing body (4), a reservoir (2) for holding a liquid, a membrane unit (3), and a flow path (7). The membrane unit comprises a membrane (5) and an actuator (6). The membrane (5) is disposed in the housing body and has a plurality of apertures, wherein the membrane is disposed so that, when liquid is held in the reservoir, the liquid is supplied to a first side of the membrane. The actuator (6) is coupled to the membrane (5) for vibrating the membrane, whereby the liquid passes through the apertures and an aerosol is generated at a second side of the membrane opposite to the first side of the membrane. The flow path (7) is defined in the housing body (4) and has a first opening (8) to the outside of the housing body at one end and a second opening (9) to the outside of the housing body at another end, so that a flow is generatable in at least a first flow direction from the second opening to the first opening in the flow path upon inhalation of a user at the first opening for entraining and delivering the generated aerosol and in a second flow direction from the first opening to the second opening in the flow path upon exhalation of the user at the first opening into the flow path. The membrane unit (3) is disposed in the flow path between the first opening and the second opening. The second opening (9) is shaped to act as a flow restrictor configured to establish a flow resistance upon inhalation of the user of 160Pa ± 100Pa, preferably 160Pa ± 60Pa, more preferably 160Pa ± 30Pa, and a flow resistance upon exhalation that is at least 25% lower than the flow resistance upon inhalation, measured at a peak flow of a sinus-shaped breathing pattern of two seconds of inhalation time and exhalation time, respectively, and 500 ml of tidal volume.

## Description

### Technical Field

The present disclosure relates to an inhalation therapy device for entraining and delivering a generated aerosol to a user or patient.

### Background Art

Inhalation therapy devices, also known as (inhalation) nebulisers or aerosol delivery devices, are widely used to deliver therapeutically effective amounts of pharmaceuticals, such as drugs and vaccines, in an aerosol form to users through their respiratory system. Inhalation therapy devices can also be used for diagnostic purposes using radioisotopes for lung challenge tests. For therapy, aerosol inhalation is the preferred root of administration for some pharmaceuticals, which can be intended for treatment of systemic or respiratory diseases.

To achieve the intended treatment, aerosol particles must be deposited in specific parts of the respiratory tract of the user's body, such as the lung. Different size particles tend to deposit in different parts of the respiratory system. It is commonly known in this field that particles having a MMAD (Mass Median Aerodynamic Diameter) of less than Spm or even less than 4µm and at least 1µm are required to allow deposition in the intended part of the respiratory system.

The aerosols for therapeutic purposes are generated and delivered to a desired location within the user's body, especially its respiratory tract, with the inhalation therapy device. To do so, a fluid or liquid, i.e., a medicament, a drug, a vaccine, etc., to be aerosolised or nebulised is supplied to a reservoir for holding the fluid or liquid in the inhalation therapy device. For aerosolising or nebulising the liquid in the reservoir, inhalation therapy devices may, for example, comprise a membrane unit.

Such membrane units comprise a membrane having a plurality of apertures and an actuator that is directly or indirectly, via a support plate supporting the membrane, coupled to the membrane for vibrating the same.

The fluid or liquid may be in contact with a first side of the membrane via gravitational force. The fluid or liquid (i.e., the medicament) passes through the apertures from the first side of the membrane and an aerosol is generated at a second side of the membrane opposite to the first side of the membrane upon vibrating the membrane.

The aerosolised or nebulised fluid or liquid, i.e., the aerosol, inside the inhalation therapy device is then supplied to the user's respiratory system within the bounds of an inhalation therapy upon inhalation of the user through the inhalation therapy device. Meaning, upon the application of a suction force by the user's breathing, especially his or her inhalation, through the inhalation therapy device, the aerosol is at least partially transported (by the suction force) from the interior of the inhalation therapy device to the user's respiratory tract.

An example for a conventional inhalation therapy device having such a configuration is, for example, derivable from DE 199 53 317 C1. The membrane unit of the disclosed inhalation therapy device comprises a cylindrical liquid reservoir container, which is delimited at one end face by a membrane having the shape of a circular disc. A liquid disposed in the reservoir contacts the side of the membrane facing the reservoir.

DE 199 53 317 C1 further discloses a membrane that is connected (welded) to a substrate (support plate) and an oscillating generator, for example, a piezo crystal, which surrounds the membrane in a circular manner and is connected (glued) to the substrate, such that the membrane can be caused to oscillate by means of the oscillating generator and an electric drive circuit.

Thereby, the liquid applied to the membrane on the first side of the membrane is conveyed through the apertures in the oscillating membrane to the second side of the membrane opposite to said first side of the membrane to be emitted into a chamber as an aerosol. The disclosed inhalation therapy device further embodies a mouthpiece or a mask connected to an outlet of the chamber and having an exhalation valve. Upon exhalation of the user, the exhaled flow will, thus, not or only to a limited extend enter the chamber and rather be expelled through the exhalation valve into the environment of the device.

The disclosed device continuously drives the piezo crystal throughout the (single) treatment time (single administration interval), in which substantially the entire liquid in the reservoir is aerosolised by the membrane. During exhalation of the user, the generated aerosol is temporarily stored in the chamber forming an aerosol cloud (bolus), which, upon inhalation, is inhaled and transported to the user's respiratory tract for therapy. Therefore, the treatment time can be kept relatively short.

One potential drawback is, however, that with an increasing output rate of the aerosol generator (membrane, piezo crystal, drive circuit, etc.) the amount of aerosol deposited on the inner walls of the device (known as a so called "rainout"), particularly in the chamber, increases. This leads to an increased loss of administrable aerosol/pharmaceutical.

Other conventionally known inhalation therapy devices drive the piezo crystal only during inhalation and stop driving the piezo crystal during exhalation. Those inhalation therapy devices are often referred to as breath-triggered devices or breath-actuated devices. It has been found that such inhalation therapy devices often have the issue of a long treatment time, i.e., a long therapy duration (long administration interval), which can be annoying to the user and, thereby, provoke a lack of user adherence, e.g., an interruption or a premature end of the therapy before the intended dose of fluid or liquid in the reservoir has been nebulised and delivered to the user's respiratory tract for therapy.

Both of the conventional inhalation therapy devices described above are oftentimes operated in such a manner that the user merely inhales through the inhalation therapy device (an inhalation flow is generated through a flow path of the device), and the inhalation therapy device is removed from the user's mouth and/or nose upon exhalation. While both enables a resistance-free exhalation of the user, the overall treatment cannot be satisfactorily monitored, as no data, such as a flow parameter in the flow path of the device, can be obtained during the exhalation. Additionally, and in case of a continuous operation, generated aerosol gets lost without any therapeutic effect for the user.

On top of that, removing the inhalation therapy device with every exhalation negatively affects the user's "natural" respiration, i.e., his or her steady breathing cycle.

Hence, there remains a need for a simple and effective inhalation therapy device that enables a short overall treatment/therapy time (single administration interval).

In some cases, a single administration interval may include at least five breathing cycles, which include five inhalations and five exhalations, and uses at least 0.25mL fluid or liquid. In other cases, a single administration interval may include at least ten breathing cycles, which include ten inhalations and ten exhalations, and uses at least 0.5mL fluid or liquid.

### Summary of the Disclosure

In view of the aforesaid, it is an object of the present disclosure to establish an inhalation therapy device that increases the overall quality of drug delivery while keeping the overall therapy time as short as possible.

This object is solved by means of an inhalation therapy device according to independent claim 1. Distinct embodiments are derivable from the dependent claims.

According to the first aspect of the present disclosure, an inhalation therapy device comprises a housing body, a reservoir for holding a liquid, a membrane unit, and a flow path.

The membrane unit is arranged inside the housing body and comprises a membrane, which is disposed in the housing body in such a manner that, when a fluid or liquid (i.e., a medicament) to be aerosolised or nebulised is held in the reservoir of the inhalation therapy device, the liquid is supplied to a first side of the membrane.

The membrane unit also comprises an actuator, which is coupled directly or indirectly via a support plate supporting the membrane, to the membrane for vibrating the same. To generate the aerosol, the membrane comprises a plurality of apertures, wherein, upon vibrating of the membrane, the liquid passes through said apertures and an aerosol is generated at a second side of the membrane, which is opposite to the first side of the membrane.

The flow path itself is defined in the housing body and comprises a first opening to the outside (environment) of the housing body (inhalation therapy device) at one end of the flow path and a second opening to the outside of the housing body at another end.

The membrane unit is disposed in the flow path between said first opening and second opening. This configuration is not limited to an inline arrangement of the membrane unit in the flow path itself. For example, the membrane unit can also be arranged in a separate channel being in fluid communication with the flow path, such that the generated aerosol can equally be transmitted to the flow path. An example for such a configuration is a T-connection inside the flow path. At the same time, it is possible to arrange the membrane unit in such a manner directly/inline in the flow path that it can at least be partially surrounded by the flow in the flow path. In other words, an envelope flow is generated about the circumference or part of the circumference of the membrane.

A flow in the flow path is, hence, generatable in at least a first flow direction from the second opening to the first opening upon inhalation of the user at the first opening for entraining and delivering the aerosol generated at the second side of the membrane inside the flow path to a user or a patient.

The "first opening" may, for example, be understood as a "mouthpiece", an inhalation mask, or the like, or at least a connection to such elements through which the user can apply a suction force to the flow path of the inhalation therapy device.

Accordingly, to establish said flow through the flow path in the first flow direction, air must accordingly be able to enter the flow path at the second opening. Accordingly, the "second opening" at least acts as an "entrance" for air when an inhalation of the user at the first opening of the therapy device is performed to entrain and deliver the generated aerosol inside the inhalation therapy device. During an inhalation phase, the second opening may, thus, also be referred to as "inlet opening" in the first flow direction and the first opening may, thus, also be referred to as "outlet opening" in the first flow direction.

The first flow direction may, in view of the above, be interpreted as an "inhalation direction" of the (air) flow through the inhalation therapy device.

It may also be possible to generate a flow in the flow path in an opposite, second flow direction from the first opening to the second opening upon exhalation of the user or patient at the first opening into the flow path for expelling the air from user's respiratory tract. The second flow direction may, thus, be interpreted as an "exhalation direction" of the (air) flow through the inhalation therapy device. During an exhalation phase, the second opening may, thus, also be referred to as "outlet opening" and the first opening may, thus, also be referred to as "inlet opening".

This enables that the inhalation therapy device can stay in contact with the user (for example, his or her mouth, and thereby his or her respiratory tract) during the whole therapy duration (treatment time) of a single administration interval and the inhalation and the exhalation can be performed through the air flow of the inhalation therapy device.

The second opening of the inhalation therapy device, according to the first aspect, is shaped to act as a flow restrictor.

To do so, the second opening is shaped in such a manner that it establishes a flow resistance upon inhalation of the user of 160Pa ± 100Pa and a flow resistance upon exhalation that is at least 25% lower than the flow resistance upon inhalation, measured, respectively, at a peak flow of a sinus-shaped breathing pattern of two seconds of inhalation time, two seconds of exhalation time, and 500ml of tidal volume.

Preferably, the flow resistance upon inhalation of the user generated by the shape of the second opening is in a range of 160Pa ± 60Pa.

More preferably, the flow resistance upon inhalation of the user generated by the shape of the second opening is in a range of 160Pa ± 30Pa.

Accordingly, the "flow resistance upon inhalation" may also be understood as "an inhalation resistance" (or a "pressure loss") against which the user has to breath when performing an inhalation through the inhalation therapy device. This may, hence, also be understood as a negative pressure inside the inhalation therapy device established via the shape of the second opening of the flow path of the inhalation therapy device. Similarly, the "flow resistance upon exhalation" may also be understood as "an exhalation resistance".

Measuring a "peak air flow" of a breathing pattern is the standardised way of evaluating air flows of anaesthetic and respiratory equipment, such as nebulising systems and components thereof. To do so, it is common practice to apply the above-mentioned sinus-shaped breathing pattern of two seconds of inhalation time, two seconds of exhalation time, and 500ml of tidal volume at anaesthetic and respiratory equipment to establish comparative information between such anaesthetic and respiratory equipment. The details of how these peak air flow values are to be detected/measured, the apparatuses required to do so, as well as the experimental conditions are, for example, reflected in the "DIN standards committee rescue services and hospital" standard DIN ISO 27427.

This international standard was developed to cover "general purpose" inhalation therapy devices and is based on adult test parameters for evaluating the suitability of respective inhalation therapy devices' intended used as disclosed by their manufacturers and to evaluate their safety as well as their compatibility between the materials of the components of the inhalation therapy devices and the dispensed liquids.

The flow resistance is basically exclusively governed by the second opening acting as the flow restrictor. This is because all cross-sections inside the flow path inside the inhalation therapy device are commonly drastically higher than the one of the second opening acting as the flow restrictor (at least) upon inhalation of the user. It is, therefore, justified to assume that the second opening acting as the flow restrictor is responsible for almost 100% of the flow resistance, i.e., the entire flow resistance inside the inhalation therapy device, as potential fluid-dynamic effects of other cross-sections inside the flow path of the inhalation therapy device are (commonly) negligible due to being larger than the cross-section of the second opening.

Based on the above-explained standard DIN ISO 27427 and based on the fact that all remaining (flow) cross-sections in the device besides the second opening (acting as the flow restrictor) are sufficiently large, such that the absolute dynamic pressures are not larger than the inhalation resistance, it is justified to consider that at the above-mentioned "peak flow" during the application of a standard (experimental) sinus-shaped breathing pattern of two seconds of inhalation and exhalation time, respectively, and a tidal volume of 500ml, to the inhalation therapy device, as the maximum absolute pressure values that are detected inside the inhalation therapy device during said application of said sinus-shaped breathing pattern.

Accordingly, this peak flow corresponds to a flow rate of 23.56 l/min through the inhalation therapy device and a standard setup using a differential pressure sensor with one sensor port inside the device (measuring static pressure) and one sensor port in the environment (measuring reference pressure) may be used to determine the pressure values inside the inhalation therapy device upon application of the above-mentioned breathing pattern and, thereby, the flow resistance.

In this connection, conventional inhalation therapy devices either enable a similar inhalation and exhalation flow resistance only or require more complex arrangements with additional structural elements to aim for a shortened single administration interval. Having increased, but identical flow resistances in the inhalation and the exhalation direction through the inhalation therapy device negatively affects the inhalation due to increased breathing effort, faster exhaustion, or dyspnoea, and a rather short inhalation time, while also negatively affecting the exhalation due to an undesired prolonged exhalation time against a noticeable exhalation resistance.

While the prolonged exhalation time and/or noticeable exhalation resistance is oftentimes leading to an undesired feeling for the user due to breathing out against a resistance, the rather short inhalation time negatively affects the therapeutical efficiency in the respiratory tract of the user.

Accordingly, the present disclosure suggests providing a second opening that acts as the flow restrictor configured to establish the above-explained flow resistance upon inhalation of the user to arrive at an inhalation therapy device that can, on the one hand, extend the inhalation time, while keeping the exhalation resistance low and thereby the exhalation time short due to a reduced "exhalation resistance".

Increasing the inhalation resistance and decreasing the exhalation resistance at the same time, offers four main advantages:
i) The maximum flow rate during inhalation is limited due to the increased breathing resistance (caused by the second opening acting as a flow restrictor) leading to higher drug deposition in the lungs. Accordingly, the drug deposition amount in the user's lung can be increased, which allows for a reduced amount of medical compound that needs to be aerosolised for therapy. Ultimately, this enables a shortened overall therapy time.
ii) The duration of the inhalation is prolonged leading to a longer (time) window where aerosol can be produced and, ultimately, to shorter therapy durations.
iii) While the inhalation is prolonged due to an increased breathing resistance, exhalation is shortened due to a decreased breathing resistance. This reduces the breathing effort, as the user is enabled to maintain breathing frequency and minute volume.
iiii) The inhalation pressure signal generally plays a more important role in turning on the membrane unit (i.e., the nebulizing unit). Having an increased absolute pressure during inhalation by the inventive shape of the second opening facilitates triggering and leads to improved therapy, especially an improved reliability and a shorter therapy duration.

In other words, the prolonged inhalation time caused by the above-mentioned inhalation resistance range advantageously affects the therapy success, as a longer and slower (in terms of flow rate) inhalation of the user through the inhalation device can be achieved, such that the aerosolised liquid (i.e., the pharmaceutical) can be supplied to the respiratory tract of the user in an improved manner. This is because a delivered dose rate (per inhalation and inhalation treatment) can be increased.

The inhalation therapy device also enables to be continuously held onto the user's mouth and/or nose during therapy (single administration interval) and both the inhalation and the exhalation are performed through the flow path of the inhalation therapy device.

It should also not be ignored having a flow restrictor directly at an opening, here the second opening, of the flow path leads to an improved cleanability and disinfection of the inhalation therapy device. This is because a flow restrictor being arranged between the first and the second opening could lead to an increased contamination in the resulting undercuts. This is the case, as such a flow restrictor would fluid-dynamically and, of course, also geometrically separate one large cavity into two smaller ones while provokes said undercuts.

According to a second aspect, the inhalation therapy device additionally comprises a sensor. The sensor is comprising a measurement port, which is arranged in the flow path. The sensor is configured to detect a flow parameter of the flow in the flow path via the measurement port.

In a preferred configuration thereof, the detected or measured "flow parameter" is a pressure value, a volume flow rate, or the like.

Any of these flow parameters can be used to trigger the aerosol production inside the inhalation therapy device's flow path via the membrane unit.

The arrangement of the measurement port in the flow path also enables to precisely evaluate at least the quality of inhalation and, thereby, enables to conclude whether, for example, the inhalation is performed long enough or should be further extended. Equally, a flow not only in the first flow direction, but also in the second flow direction, i.e., an exhalation of the user through the inhalation therapy device, may be evaluated as well. This evaluation enables an optimized activation and/or a so called "beath triggering" of the membrane unit of the inhalation therapy device.

Preferably, the measurement port is disposed upstream of the membrane of the flow path when seen in the first flow direction. In other words, the measurement port is arranged "behind" the membrane unit when the inhalation therapy device is held by a user and/or is used for inhalation therapy. The measurement quality can be improved therewith, as it can be effectively prevented that an undesired amount of aerosol/fluid particles, sputum, and/or bacteria stemming from the user are brought in contact with the measurement port.

According to a third aspect, the flow path is shaped in such a manner that, upon inhalation, the entire flow volume along the first direction to the first opening passes through the second opening.

In this connection, the "flow volume along the first direction" is to be understood as the volume, which is breathed on by the user upon inhalation through the inhalation therapy device and is accordingly moved in the inhalation therapy device and out of it into the user's respiratory tract. However, it is, for the sake of completeness, noted that not all the volume entering the inhalation therapy device through the second opening is reaching the user's respiratory tract due to the volume of the inhalation therapy device itself.

This enables reducing the structural complexity inside the housing body, as any sub-paths, measurement paths, or the like can be omitted. Accordingly, the device can be disinfected and/or cleaned more easily.

According to a fourth aspect, the second opening is arranged upstream of the membrane unit when seen in the first flow direction.

That is, the second opening may be understood as being arranged "behind" the membrane unit, when the inhalation therapy device is used for therapy, i.e., when air is sucked into the respiratory tract of the user via the first opening of the flow path. This enables to arrange the second opening far away from the first opening, which is arranged downstream of the membrane unit when seen in the first flow direction. Accordingly, an air flow is guided from "behind" the membrane unit when seen in the first flow direction from the second opening through the flow path, the air then passes the membrane unit, for example, by being guided around it, merges with the generated aerosol by its flow around and/or in the region of the second side of the membrane of the membrane unit and is then guided through the flow path towards the first opening.

According to a fifth aspect, the second opening is arranged at an upper half of the housing body, when the inhalation therapy device is held by a user during therapy. The therapy is, for example, to performed in a sitting position of the user, when a head of the user is in an upright position.

This enables to prevent that, when the inhalation therapy device is held by a user during therapy, the second opening through which air enters the flow path is accidentally blocked by the user holding the inhalation therapy device during therapy in a sitting body position having his or her head in an upright position. It can, hence, be assured that the detection of the flow parameters, for example, the measurement of pressure values inside the flow path, can be reliably performed during therapy. Vice versa, it can be ensured that the second opening is permanently open and does not get blocked accidentally.

Preferably, the second opening is arranged not only at an upper half of the housing body but also in an area that is in a range of 5mm to 10mm away from an uppermost position of the housing body, when the inhalation therapy device is held by the user during therapy in the sitting body position having his or her head in an upright position.

Accordingly, the risk of accidentally blocking the second opening during therapy can successfully be reduced. This is the case, as it is highly implausible that the second opening being arranged at an uppermost position of the housing body gets blocked, for example, by a user's hand or fingers holding the inhalation therapy device.

The term "uppermost position" is, in this connection, to be understood as referring to the housing body only. Accordingly, any additional structural elements of the inhalation therapy device, such a lid for covering the reservoir, shall not be taken into consideration.

Alternatively, the term "uppermost position" is to be understood as referring to the flow path in the inhalation therapy device only, when the inhalation therapy device is held by a user during therapy in the sitting body position having his or her head in an upright position.

According to a preferred embodiment, the inhalation therapy device may additionally comprise a handle portion for enabling the user to comfortable hold the inhalation therapy device. The holding portion may be reversibly attachable and detachable to a lower portion of the housing body, which may be understood as an aerosolization portion in this regard. Accordingly, the second opening is arranged in the aerosolization portion at a position that is distant, preferably most distanced, from the handle portion. This, again, enables to prevent that the second opening can, for example, be blocked by a user's hand or fingers during therapy with the inhalation therapy device.

In a further embodiment, the second opening may be shaped as a splitting channel to the housing body, i.e., the outer shell of the inhalation therapy device to more reliably omit a blocking by the user or the user's hand/fingers during (inhalation) therapy with the inhalation therapy device. The splitting channel may comprise ribs, partitions, or fins that extend towards the housing body to form the outer shell of the device at the position of the second opening in the housing body.

According to a sixth aspect, the cross-sectional shape of the second opening gradually decreases, preferably continuously decreases when seen along the second flow direction from the first opening to the second opening.

This is an exemplary shape of the second opening of how to achieve the desired inhalation flow resistance of 160Pa ± 100Pa, preferably 160Pa ± 60Pa, more preferably 160Pa ± 30Pa. This enables to prolong the inhalation time and, thereby, improves the application of the aerosolised fluid or aerosolised liquid (i.e., the medicament) to the user's respiratory tract.

The gradually, preferably continuously, decreasing of the second opening's cross-section along the second flow direction provokes that air that enters the flow path via the second opening upon inhalation is confronted with a narrowed cross-sectional shape and an arres, i.e., a sharp edge at the outermost position of the second opening. This shape provokes a flow separation at the outermost position of the second opening and thereby leads to an increased flow resistance upon inhalation due to the urge of increasing the velocity. At the same time, the decreasing cross-sectional shape along the second flow direction provokes a reduced exhalation resistance due to the lack of sharp edges and, consequently, avoids flow separation along the flow along the second flow direction.

This leads to a facilitated exhalation through the inhalation therapy device and, thereby, a more pleasant feeling for the user, as the exhaustion of air does not have to be performed against a large resistance. Accordingly, the inhalation time can be prolonged, and the exhalation time can be shortened, resulting in a high duty cycle resulting in a short overall therapy time.

The "gradually decreasing cross-sectional shape" along the first flow direction may, for example, be understood as a conical shape of the second opening with a narrower cross-section to the outside of the housing body than inside. Yet, the term is not limited to such shapes.

The second opening of the flow path may be configured as a separate structural element delimiting the flow path inside of the housing body to the outside or may be shaped as an integral portion of the housing body. Irrespective thereof, it is possible that the outermost position of the second opening protrudes from the housing body or is arranged in such a manner that it is arranged offset to the inside leading to a uniform outer shape of the housing body.

According to a seventh aspect, the end of the second opening that is oriented towards the outside of the housing body comprises an oval, an elliptical, a polygonal, or a circular cross-sectional shape.

Preferably, the second opening comprises a curved outer shell in the backpart, i.e., in the region of the second opening that is oriented to the inside of the inhalation therapy device.

As mentioned above, this configuration is advantageous for achieving at least the desired inhalation flow resistance of 160Pa ± 100Pa, preferably 160Pa ± 60Pa, more preferably 160Pa ± 30Pa, while establishing a form that suits the remaining shape of the inhalation therapy device and a reduced exhalation resistance.

According to an eight aspect, the end of the second opening that is oriented towards the outside of the housing body comprises chevrons.

In other words, the second opening that is oriented towards the outside of the housing body is shaped like a chevron nozzle.

Chevrons are, for example, well-known in the aerospace (and wind-energy) industry as "saw-tooth" patterns on the trailing edge of jet engine nozzles (or windmills) that are used for noise reduction. For example, in aerospace turbines, the sharp edges enable a smooth mixing of hot air of the engine core and cooling air flowing through the engine fan, which reduces noise creating turbulences.

In line therewith, also the second opening of the flow path through the housing body of the inhalation therapy device may be provided with such chevrons, i.e., saw-tooth patterns on the end of the second opening that is oriented towards the housing body to reduce any noises due to the air flow, such as whistling, hissing, etc. Consequently, the chevrons enable a reduction of background noises during therapy and, thereby, lead to an improved application of the inventive inhalation therapy device.

In a preferred embodiment, the chevrons, preferably at least three chevrons, more preferably six chevrons, are evenly distributed around the circumference of the end of the second opening that is oriented towards the outside of the housing body.

According to a ninth aspect, an inhalation therapy device according to any of the first to seventh aspects may additionally comprise an elastic protective member, which is arranged at the end of the second opening that is oriented towards the outside of the housing body. In this connection, the elastic protective member is shaped in such a manner that the second opening remains open even upon inhalation and/or exhalation of the user through the inhalation therapy device.

The elastic protective member may have an appropriate elasticity to successfully prevent the potentially protruding second opening, and its potentially protruding sharp edge to the outside of the flow path, of any damage when the inhalation therapy device is accidentally dropped or exposed to other kinds of impacts or stresses.

At the same time, the elasticity is chosen in such a manner that the elastic protective member is unable to cover the second opening in its entirety during inhalation/exhalation. That is, the elastic protective member is not to be understood as a duckbill valve, or the like, that would lead to a closing of the second opening upon inhalation of the user at the first opening. To the contrary, the elastic protective member is shaped in such a manner, that the second opening remains open at any time, i.e., even upon inhalation and/or exhalation of the user through the inhalation therapy device. Consequently, the elastic protective member is rather a protective member for protecting the second opening from any damages due to external forces and, at the same time, does not affect the achievable flow resistance that is established by the shape of the second opening itself.

Yet, in another configuration, the deflection of the elastic protective member during inhalation (and exhalation) might help to achieve the desired flow resistance during inhalation (and exhalation) by increasing or decreasing the cross-section at the end of the second opening that is oriented towards the outside of the housing body accordingly.

Specifically, the flexibility of the elastic protective member at the second opening may also support to generate an inhalation resistance that deviates from the exhalation resistance. Upon inhalation, the flexible member of the second opening bends inward, only partly closing the second opening and, therefore, increasing the inhalation resistance. During exhalation, the elastic protective member bends outwards due to its flexibility, giving the exhalation flow more space, which results in lower velocities at the second opening and, hence, reduced exhalation breathing resistances. This embodiment may, therefore, also be understood as functioning like a cut-off duckbill valve.

The elastic protective member may, for example, be made of TPU (= thermoplastic polyurethan).

In a preferred configuration, also the elastic protective member comprises chevrons.

According to an alternative configuration of the ninth aspect, an inhalation therapy device according to any of the first to seventh aspects may additionally comprise a protective member, which is arranged at the end of the second opening that is oriented towards the outside of the housing body. In this connection, the protective member is shaped in such a manner that the second opening remains open even upon inhalation and/or exhalation of the user through the inhalation therapy device, and the protective member is configured as a plurality of fins, ribs, or chevrons.

In line with the configuration above, this enables to successfully prevent the potentially protruding second opening, and its potentially protruding sharp edge to the outside of the flow path, of any damage when the inhalation therapy device is accidentally dropped or exposed to other kinds of impacts or stresses.

According to a tenth aspect, the flow path is shaped in such a manner that upon inhalation, the entire flow volume along the first direction passes through the second opening and, upon exhalation, the entire flow volume along the second direction also passes through the second opening.

Accordingly, the air that should be used for therapy is completely introduced into the flow path towards the first opening for entraining and delivering the generated aerosol through the second opening and is, at the same time, also exhausted from the inhalation therapy device via the second opening. Hence, the inhalation therapy device comprises only one single flow path "channel", through which the entire inhalation flow for therapy passes. Also in this connection, it is, for the sake of completeness, once again noted that not all the volume entering the inhalation therapy device through the second opening is reaching the user's respiratory tract due to the volume of the inhalation therapy device itself, i.e., due to its dead space.

This allows to omit the requirement of having more than one channel, in which various relevant elements, sensors, etc. are arranged.

In this connection, the second opening is shaped as a fixed geometry passive valve. The "fixed geometry passive valve" is to be understood as a geometrical structure that does not require the control and/or presence of any moving elements that need to be operated for allowing air to be introduced into the flow path and being exhausted from the flow path upon exhalation.

The second opening being shaped as a fixed geometry passive valve is configured to generate a flow resistance upon inhalation in the first flow direction that is at least 25%, preferably 50%, more preferably 75%, most preferably 90% higher than upon exhalation in the second flow direction.

Increasing the inhalation (flow) resistance and decreasing the exhalation (flow) resistance at the same time, offers four main advantages:
i) The maximum flow rate during inhalation is limited due to the increased breathing resistance caused by the second opening acting as a flow restrictor leading to higher drug deposition in the lungs. Accordingly, the drug deposition amount in the user's lung can be increased, which allows for a reduced amount of medical compound that needs to be aerosolised for therapy. Ultimately, this enables a shortened overall therapy time.
ii) The duration of the inhalation is prolonged leading to a longer (time) window in which aerosol can be produced and, ultimately, to a shorter therapy duration.
iii) While the inhalation is prolonged due to an increased breathing resistance, exhalation is shortened due to a decreased breathing resistance by the fixed geometry passive valve as defined above. This reduces the breathing effort as the user is able to maintain breathing frequency and minute volume.
iiii) The inhalation pressure signal generally plays a more important role in turning on the nebulising unit. Having an increased absolute pressure during inhalation by the inventive shape of the second opening facilitates triggering the membrane unit and leads to improved therapy, especially an improved reliability and a shorter therapy duration.

In line with the aforesaid, also these values for the flow resistance in the first flow direction and in the second flow direction are measured at the peak flow of the (standard) sinus-shaped breathing pattern of two seconds of inhalation time and exhalation time, respectively, and 500ml of tidal volume.

This may, for example, be achieved by a shape in which, during inhalation, the effective cross-section of the second opening is reduced compared to a(n) (effective) cross-sectional shape of the second opening in the exhalation direction, such that an increased flow velocity in the inhalation direction and, hence, higher resistance is required which allows to achieve a hysteresis effect. Vice versa, in the exhalation direction, substantially the whole cross-section of a second opening being shaped as a fixed geometry passive valve can be used, such that the exhalation can be performed at smaller velocities and, hence, against a smaller resistance.

The shape of the second opening provoking a different inhalation resistance than an exhalation resistance may, thus, also be understood as a "fluidic diode".

Yet, the geometrical shape is not limited to said configuration. To the contrary, the effects could also be created by using a shape, in which the flow during inhalation is guided around obstacles (e.g., turns, deflections, narrows), while during exhalation, the flow can pass the section comparably smooth, i.e., with a reduce flow resistance in the exhalation flow direction. One commonly known shape of such a second opening provoking a different inhalation resistance than an exhalation resistance may be the "tesla valve".

Irrespective of the geometrical shape chosen, it is possible to achieve an inhalation therapy device, in which the inhalation flow resistance is higher than an exhalation flow resistance. This allows to achieve the above-sketched advantages.

According to an eleventh aspect, the second opening is not limited towards a configuration with one single second opening of the flow path to the outside of the housing body.

According to the eleventh aspect, the second opening of the inhalation therapy device according to the tenth aspect is formed by a plurality of sub-openings that are respectively shaped as a fixed geometry passive valve. Each of the sub-openings provides for an opening of the flow path to the outside of said housing body. Further, these sub-openings are configured to generate together a flow resistance upon inhalation in the first flow direction that is at least 25%, preferably 50%, more preferably 75%, most preferably 90% higher than upon exhalation in the second flow direction.

Splitting the configuration of the second opening into several sub-openings enables to reduce the risk of entirely blocking the second opening during the therapy, while achieving more freedom to accommodate the flow restrictor on the device. At the same time, also the provision of a second sub-opening allows to achieve an inhalation flow resistance of 160Pa ± 100a, preferably 160Pa ± 60Pa, more preferably 160Pa ± 30Pa, and, compared to the inhalation resistance, a reduced exhalation resistance which, as mentioned earlier, beneficially affects the therapy duration.

According to a twelfth aspect, the second opening of the inhalation therapy device according to the tenth or eleventh aspect is shaped in such a manner that the flow resistance in upon inhalation in the first flow direction is between 25% to 500%, preferably between 50% to 300%, more preferably between 75% to 200% higher than upon exhalation in the second flow direction.

While the inhalation flow resistance shall be kept in a range of 160Pa ± 100Pa, preferably 160Pa ± 60Pa, more preferably 160Pa ± 30Pa, this configuration and shape of the second opening results in an exhalation resistance that is noticeably lower than the inhalation resistance.

According to a thirteenth aspect, the second opening of the inhalation therapy device according to any of the tenth to twelfth aspects is shaped in such a manner that the flow resistance upon exhalation in the second flow direction does not exceed 135Pa, preferably 100Pa, more preferably 80Pa measured at the peak flow of the (standard) sinus-shaped breathing pattern of two seconds of inhalation time, two seconds of exhalation time, and 500ml of tidal volume.

Experiments and flow simulations have shown that the configuration of the above-mentioned inhalation flow resistance and said exhalation resistance of a maximum of 80Pa is the preferred range for entraining and delivering the generated aerosol to the user's respiratory tract for the above-mentioned reasons.

Given the fact that these inhalation and exhalation resistances can be achieved by the geometric shape of the second opening being shaped as a fixed geometry passive valve allow for a particularly simple, compact, yet efficient inhalation therapy device.

According to a fourteenth aspect, the inhalation therapy device according to any of the tenth to thirteenth aspects further comprises an air filter device. Preferably, said air filter device is reversibly detachable and attachable to the housing body. This air filter device is configured to filter harmful or even toxic substances from the aerosol generated inside the housing body via the membrane unit and being delivered to the user's respiratory tract while the user exhales, such that the aerosol cannot escape to the environment.

To do so, the air filter device is shaped to cover the second opening, so that upon inhalation, the entire flow volume along the first flow direction passes through the air filter device and, upon exhalation, the entire flow volume along the second flow direction passes through the air filter device as well.

In an alternative embodiment, the air filter device may be arranged in the flow path at a position upstream of the second opening (that is, the second opening could be placed downstream of the filter) when seen in the second flow direction.

Irrespective of the configuration chosen, the entire flow volume along the first direction and the second direction passes through the second opening and the air filter device. It should be noted in this scenario that the air filter device and the shape of the second opening together lead to an increased inhalation and exhalation flow resistance. Meaning, the air filter device and the second opening together are crucial for defining the flow resistance in the inhalation and the exhalation direction.

However, in a further embodiment, the resistance of the second opening during inhalation and exhalation might be adapted in order to minimize the additional breathing resistance due to the filter.

According to a fifteenth aspect, the inhalation therapy device according to any of the first to ninth aspects, further comprises a third opening to the outside of the housing body, wherein the third opening is provided in the flow path between the first opening and the second opening.

This third opening is provided with a check valve, which is configured to restrict a flow through the first opening in the first flow direction and is configured to enable a flow through the third opening in the second flow direction from the first opening to the second opening for enabling at least a partial, preferably substantially a full, expel of air through the third opening.

Such an inhalation therapy device is configured to allow an inhalation flow through the second opening, as the "check valve" at the third opening blocks said opening in the flow path upon inhalation, while, upon exhalation through the inhalation therapy device, the third opening and its check valve provide for a reduced resistance for expelling the air from the inhalation therapy device due to the opening of the check valve. As such, at least a large portion of the exhausted air can be expelled through the freely opening check valve at the third opening. Accordingly, the "check valve" may equally be interpreted as an "one-way valve", which establishes a hysteresis. The increased opening area of the second and third opening combined during exhalation leads to reduced local flow velocities at the openings at the give volume flow rates and, hence, reduced exhalation resistance.

According to a sixteenth aspect, the third opening in the inhalation therapy device according to the fifteenth aspect is provided upstream of the membrane unit of the flow path when seen along the first flow direction.

Accordingly, the third opening and its check valve can be arranged at a position that is sufficiently far away from the first opening encountering the user's mouth, or mouth and nose, such that unpleasant feelings from the air being exhausted through the inhalation therapy device can be avoided.

According to a seventeenth aspect, the inhalation therapy device according to the fifteenth or sixteenth aspect further comprises the air filter device, which, preferably is reversibly detachable and attachable to the housing body. This air filter device is configured to filter harmful substances from the aerosol. Further, the air filter device is shaped to cover the third opening, preferably the second opening and third opening, such that the flow in the second flow direction passes through the air filter device through the outside of the housing body upon exhalation.

In an alternative embodiment, the air filter device may be arranged in the flow path at a position upstream of the second and third opening (that is, the second and third opening can be placed downstream of the filter) when seen in the second flow direction.

Such configurations enable that the inhalation flow from the second opening to the first opening, i.e., the inhalation flow direction through the housing body for entraining and delivering the generated aerosol, does not have to pass the air filter device. At the same time, the safety of the environment during therapy can be ensured by the covering of the third opening and its check valve with the air filter device, such that the main flow in the second flow direction can be filtered. Thus, it can be avoided that large amounts of toxic substances are expelled from the inhalation therapy device to the environment.

### Brief Description of the Drawings

Hereinafter, non-limiting examples of the disclosure are explained with reference to the drawings, in which:
Fig. 1 shows an embodiment of the inhalation therapy device according to the present disclosure in an perspective view.
Fig. 2 shows a schematic cross-sectional view along the flow path of the inhalation therapy device of Fig. 1.
Fig. 3 illustrates an exemplary configuration of the second opening of the inhalation therapy device.
Fig. 4 shows another exemplary configuration of the second opening of the inhalation therapy device.
Fig. 5 shows an even further exemplary configuration of the second opening of the inhalation therapy device.
Fig. 6 illustrates the inhalation flow into the second opening of the inventive inhalation therapy device.
Fig. 7 shows the exhalation through the inhalation therapy device and its second opening.
Fig. 8 illustrates an exemplary configuration of the second opening with a protective member.

### Detailed Description of Preferred Embodiments

Currently preferred embodiments of the present disclosure will now be described with reference to the accompanying drawings. The preferred embodiments refer to an inhalation therapy device 1.

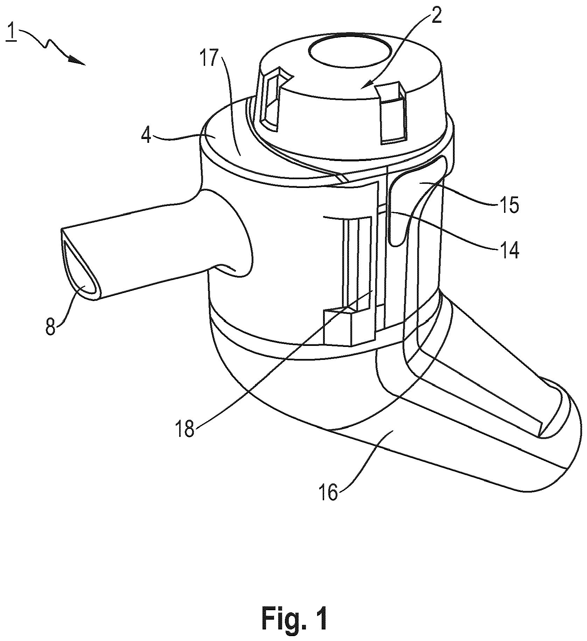

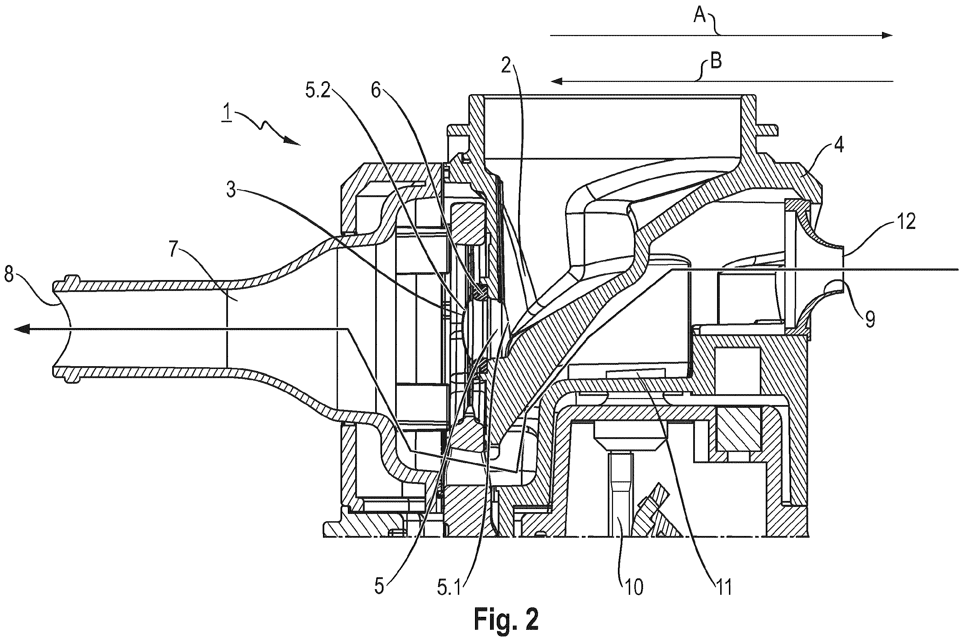

In the following a preferred embodiment of the inhalation therapy device 1 of the present disclosure will be described with reference to Figs. 1 and 2.

It is to be understood that such inhalation therapy devices 1 are oftentimes also called "aerosol delivery devices" or "nebulisers", for example, for a therapeutic use in/via a user's respiratory system.

Fig. 1 shows an exemplary perspective view of the inhalation therapy device 1 according to a currently preferred embodiment of the present disclosure.

It is derivable therefrom, that the overall shape of the inhalation therapy device 1 is split into two main parts. The inhalation therapy device 1 comprises a holding portion 16, which may also be called "controller portion", and an aerosolization portion 17, which may also be called "nebuliser portion" or "nebulisation set". Yet, the present disclosure is not limited to such a "split" configuration: it may also be possible to form the holding portion 16 and the aerosolization portion 17 as integral elements. While the holding portion 16 as illustrated in Fig. 1 aims to enable that the inhalation therapy device 1 can be held by a user during (aerosol) therapy, the aerosolization unit 17 itself is provided to aerosolise or nebulise a fluid or liquid (i.e., a medicament, a drug, an active substance, an radioactive material, or the like) via a membrane unit 3 (which is described in more detail with reference to Fig. 2 below).

The holding portion 16 may comprise the most relevant, preferably all, electronic features necessary to operate the inhalation therapy device 1 such that the aerosolization unit 17 can be configured as a throw-away product or a product, which is to be used for a certain amount of time, such as a certain number of therapies, weeks, or months. At the same time, the holding portion 16 comprising all relevant electric and sensory elements for operating the inhalation therapy device 1 and monitoring the therapy process may be construed as a long-lasting element, which nearly does not need to be cleaned, or specifically disinfected before every therapy session. For example, to clean the holding portion 17, a wet cleaning tissue is sufficient.

To enable a decent cleanability of the aerosolization unit 17 and in easy access of the membrane unit 3 arranged inside the aerosolization unit 17, the exemplary embodiment according to Fig. 1 provides a latch 18. The latch 18 enables access to the interior of the inhalation therapy device 1, especially to the membrane unit 3 inside the aerosolization unit 17 of the inhalation therapy device 1.

In the technical field given, such membrane units 3 are also known as "head units". Similarly, the aerosolization unit 17 is known as a "nebset", which reflects an abbreviation of nebulisation set.

The preferred embodiment illustrated in Fig. 1 is an inhalation therapy device 1 through which a user inhales and exhales during the therapy. That is, the inhalation therapy device 1 is brought into contact with the user's mouth, for example, via a mouthpiece, or is brought into contact with the user's mouth and nose, for example, via a face mask, such that the regular inhalation and exhalation of the user is performed through the inhalation therapy device 1. That is, the inhalation therapy device 1, specifically its first opening 8 (which will be described in more detail below) shall not be removed from the user's mouth (mouthpiece), or mouth and nose (facemask) during the aerosol therapy.

How the aerosol is generated and how the actual therapy is to be performed inside the inhalation therapy device 1 will now be described in more detail with reference to Fig. 2.

Fig. 2 shows a cross-sectional view along a flow path 7 through the inhalation therapy device 1 and, in this connection, focusses on the (upper) aerosolization unit 17 of the inhalation therapy device 1 of Fig. 1. Accordingly, merely the uppermost section of the holding portion 16, when the inhalation therapy device 1 is held in an ideal position by a user during therapy in a sitting position of the user with an upright position of the user's head, is illustrated in Fig. 2.

The circumference of the inhalation therapy device 1, especially of its aerosolization unit 17, is defined by a housing body 4.

Here and in the following, it shall be understood that Fig. 2 shows the orientation of the inhalation therapy device 1 when it is held by a user in an ideal position during therapy in a standing or sitting position of the user.

In this scenario, a reservoir 2 for holding a fluid or liquid (i.e., the medicament) is provided at an upper section of the housing body 4. It is apparent from Fig. 1 that a lid is provided on the upper section of the reservoir 2 for ensuring that the fluid or liquid in the reservoir 2 is not spilled during therapy. Yet, in Fig. 2, said lid is not illustrated for easier orientation.

Additionally, it is derivable from Fig. 2 that also a membrane unit 3 is provided in the housing body 4.

To produce the aerosol for therapy, the membrane unit 3 comprises a membrane 5 and an actuator 6. In the cross-sectional view Fig. 2, the membrane 5 is arranged in an upright position, i.e., in a vertical position, in the housing body 4, such that the membrane 5 can encounter the fluid or liquid being held in the reservoir 2. In other words, the reservoir 2 and the membrane 5 are disposed adjacent to each other, such that the liquid can be supplied to a first side 5.1 of the membrane 5 when a fluid or liquid is held in the reservoir 2. Doing so enables that the fluid or liquid is automatically, i.e., gravitationally, transported to the membrane 5 of the membrane unit 3, by the gravitational force, for producing aerosol for the user's therapy. This is achieved by arranging the membrane 5 at a lowermost position of the reservoir 2 when the inhalation therapy device 1 is held by a user during therapy (see Fig. 2).

The membrane 5 comprises a plurality of apertures and the actuator 6 is coupled to the membrane 5, such that it can vibrate the same. By doing so, the liquid passes through the apertures, and an aerosol is generated at a second side 5.2 of the membrane 5 opposite of the first side 5.1 of the membrane 5. That is, an aerosol is generated in the chamber on the in Fig. 2 left-hand side of the membrane unit 3 inside the housing body 4, for example, upon inhalation.

To entrain and deliver the generated aerosol at the second side 5.2 of the membrane 5 inside the housing body 4 of the inhalation therapy device 1 to the user's respiratory tract, the inhalation therapy device 1 comprises a flow path 7.

Said flow path 7 comprises a first opening 8 to the outside of the housing body 4 at one end thereof and a second opening 9 to the outside of the housing body 4 at another end. In the illustrated embodiment of Fig. 2, the first opening 8 and the second opening 9 are arranged at opposing sides of the housing body 4 and may be formed as separate structural elements that form end portions of the flow path. Yet, said arrangement is not binding and it may also be possible that, for example, the position of the second opening 9 is arranged off-set from a longitudinal axis through the first opening 8 of the flow path 7 and that the first and second opening of the flow path 7 are integral parts of the housing body (having a shape that is described in more detail below).

As mentioned earlier, it is the first opening 8 that is to be brought into contact, may it be directly or indirectly, for example, via a mask, with the user's respiratory tract via his or her mouth, or his or her mouth and nose, to enable inhalation and exhalation through the inhalation therapy device 1 during the aerosol therapy.

Vice versa, the second opening 9 allows air to enter the flow path 7 and to be expelled therefrom. Hence, the flow path 7 and its first opening 8 and second opening 9 to the outside of the housing body 4 enable that a flow is generatable in at least a first flow direction A from the second opening 9 to the first opening 8 in the flow path 7 upon inhalation of the user at the first opening 8.

The membrane unit 3 is disposed in the flow path 7, and thereby in said "flow" generatable by the users breathing, especially his or her inhalation, in such a manner that it can be at least partially surrounded by the flow through the flow path 7. For example, the membrane unit 3 may be arranged in an axially and circumferentially centred position of the flow path 7, such that a sheathing enveloping flow can pass the membrane unit 3 inside the flow path 7 during inhalation, and preferably during exhalation, of the user during breathing through the inhalation therapy device 1 during therapy.

In this connection, the above-mentioned flow in the first flow direction A from the second opening 9 to the first opening 8 may also be understood as an inhalation flow (see Fig. 2). Since the exhalation of the user shall also be performed through the inhalation therapy device 1, a second flow direction B from the first opening 8 to the second opening 9 in the flow path 7 upon exhalation of the user at the first opening 8 is generatable as well, which may be understood as an "exhalation flow".

Fig. 2 illustrates the first flow direction A and the second flow direction B. On top of that, the curved arrow of Fig. 2 extending from the second opening 9 to the first opening 8 represents an exemplary flow path 7 of the air along the first flow direction A (i.e., an inhalation air flow).

Since the membrane unit 3 inside the flow path 7 is arranged to be at least partially, preferably entirely surroundable by an air sheath air flow upon inhalation of the user through the inhalation therapy device 1, the aerosol generated at the second side of the membrane can merge with the air guided from the second opening 9 through the flow path 7 along the first flow direction A in the area in front the second side 5.2 of the membrane 5 and can, accordingly, be "transported" to the first opening 8 for entraining and delivering the generated aerosol to the respiratory tract of the user.

This is the case, as the user's inhalation at the first opening 8 establishes the above-mentioned "flow" due to suction force to the air being inside the flow path 7 and air getting sucked into the flow path 7 via its second opening 9.

To evaluate the quality of the inhalation therapy, it is required to observe the way the inhalation and the exhalation through the inhalation therapy device 1 is performed. In this connection a "good therapy" equals a therapy, in which among other parameters, the inhalation flow rate is low and the tidal volume is high. A low inhalation flow rate can be achieved by increasing the flow resistance during inhalation. If the resistance during inhalation and exhalation is however increased above a certain level, this might lead to an unpleasant feeling for the user or even dyspnoea, which increases the chances that the inhalation therapy is interrupted by the user. This can be avoided if the exhalation resistance is lowered, causing the exhalation duration to be shortened and, hence, the normal minute volume of the user can be maintained. This fact increases the ratio between inhalation and exhalation duration, which leads to higher duty cycles in the operation of the membrane unit 3 and, ultimately, shorter treatment times. It also increases the signal quality of a sensor during inhalation in case a pressure sensor is used and, hence, allows for a precise triggering of the membrane unit 3. Overall, the quality of the therapy is thereby increased.

Hitherto, the preferred embodiments of the present disclosure found an easy to manufacture and disinfect solution that achieves a beneficial short overall therapy time, in which the inhalation flow rate can be limited without unpleasant resistances during the therapy.

To evaluate flow parameters at least upon inhalation of the user, the inhalation therapy device 1 comprises a sensor 10, which is configured to detect, for example, a pressure value inside the flow path 7. To do so, the sensor 10 comprises a measurement port 11 being arranged in the flow path 7. Accordingly, the sensor 10 can detect a pressure value for evaluating the flow resistance at least of the inhalation flow in the flow path 7 via said measurement port 11. As illustrated in Fig. 2, the provision of a measurement port 11 allows a distant arrangement of the sensor 10 from the measurement port 11 inside the flow path 7. As can be taken from Fig. 2's cross-sectional illustration, it is, for example, possible that the sensor 10 is arranged in the holding portion 16, whereas the measurement port 11 is arranged in the flow path 7 through the housing body 4 of the aerosolization unit 17. To enable detection of the flow pressure, the measurement port 11 and the sensor 10 may, for example, be connected, as illustrated in Fig. 2, via a hose. The pressure sensor 10 may, for example, be a differential pressure sensor that determines a static pressure inside the flow path 7 and compares the same to the environmental pressure, i.e., a reference pressure.

To achieve the desired aerosol therapy success, the inventors have detected that it is particularly an inhalation flow resistance of 160Pa ± 30Pa inside the housing body 4, which allows to achieve a sufficient resistance against the regular breathing pattern of the user, while not overwhelming the user with a too large inhalation resistance, which could potentially lead to a premature termination of the therapy.

To establish said flow resistance upon inhalation of the user, the second opening 9 is shaped to act as a flow restrictor. That is, the geometrical form of the second opening 9 acts as a "hurdle" for the air being sucked into the flow path 7 into the inhalation therapy device 1 upon inhalation of the user at the first opening 8.

At the same time, the shape of the second opening 9 acts as a less strong "hurdle" for the air being expel from the patient's respiratory tract upon exhalation into the flow path 7. Accordingly, a stronger inhalation resistance compared to the exhalation resistance can be achieved.

To precisely and comparatively measure and determine flow parameters inside the housing body 4 and its flow path 7 upon inhalation of the user, this flow measurement is to be performed via the above-mentioned pressure sensor 10 and at a peak flow value of a sinus-shaped breathing pattern of two seconds of inhalation time and two seconds of exhalation time and a tidal volume of 500ml. This is precisely defined in DIN ISO 27427.

In the exemplary embodiment of Fig. 2, not only the measurement port 11, but also the second opening 9 of the flow path 7 is arranged upstream of the membrane unit 3 when seen in the first flow direction A. In the embodiment of Fig. 2, the entire flow volume along the first flow direction A to the first opening 8 passes through the second opening 9 into the flow path 7 past the measurement port 11 and the membrane unit 3, and merges with the aerosol at the second side 5.2 of the membrane 5 before it gets expelled from the inhalation therapy device 1 into the user's body at the first opening 8 of the flow path 7.

In the embodiment of Fig. 2, also the entire flow volume along the second flow direction B passes through the second opening 9, upon exhalation.

It is, in this connection, apparent from Fig. 2 that the second opening 9 is arranged at an upper half of the housing body 4.

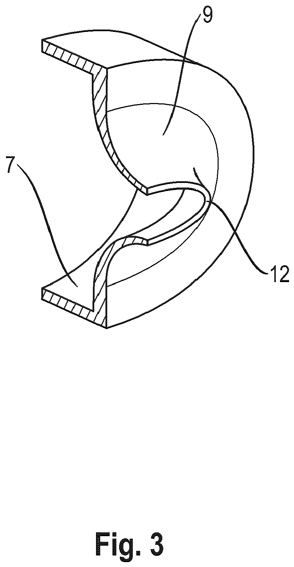

The actual shape of the second opening 9, which is responsible to consider it as a flow restrictor, will now be described in more detail with reference to Figs. 3 to 5.

To achieve the above-mentioned flow resistances via the second opening's shape, a geometrical form is to be established that provokes a higher flow resistance in the inhalation flow direction than in the exhalation flow direction. Typical relationships of the inhalation flow resistance to the exhalation flow resistance established by the second opening's shape are 125%, 150%, 175%, 200%, or even 300% or 400%. In a most preferred embodiment, the relationship of the inhalation flow resistance to the exhalation flow resistance established by the second opening's shape is 200%. Accordingly, an inhalation resistance of 160 Pa can be established while the exhalation resistance is "only" 80 Pa.

To achieve the same, the flow path's second opening 9 is shaped as a fixed geometry passive valve. The second opening 9 may be formed as an integral part of the housing body 4 or as a separate structural element being connected to the housing body 4 and forming the outlet of the flow path 7 through said housing body 4 for delivering the generated aerosol to the user during therapy.

It can be taken from Figs. 2, 3, 4, and 5 that the cross-sectional shape of the second opening 9 continuously decreases along the second flow direction B. This continuously decreasing cross-sectional shape of the second opening 9 along the second flow direction B enables that the flow resistance in the first flow direction A can be made higher than in the second flow direction B without the need of any active elements such as actuated valves, or the like. Yet, it is not necessarily required to establish said relationships of the inhalation flow resistance to the exhalation flow resistance with one single second opening 9 in the housing body 4 only. It may equally be possible that the relationship of the inhalation flow resistance to the exhalation flow resistance is achieved by a plurality of sub-openings that together form the above-mentioned flow resistances of the second opening 9. Yet, for easier orientation, it will now be focused on embodiments with one single second opening 8, as illustrated in Figs. 2 to 5.

The embodiments of Figs. 2 to 5 share a continuously decreasing cross-sectional shape along the second direction B but differ in their shape, which can easiest be seen at the end 12 of the second opening 9 that is oriented towards the outside of the housing body 4.

Dependent on the desired shape on the outside of the housing body 4 and its appearance, it is, for example, possible to form the end 12 of the second opening 9 being oriented towards the outside of the housing body 4 in an oval or elliptical cross-sectional shape. This is illustrated in Fig. 3.

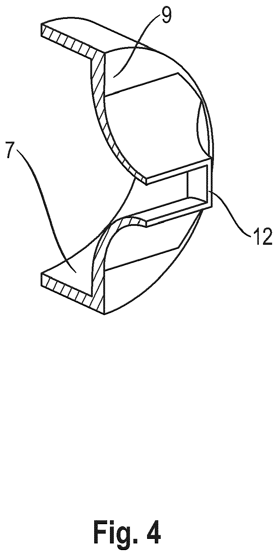

It is at the same time apparent, for example, from Fig. 4, that it is also possible to provide the end 12 of the second opening 9 with a polygonal, here a rectangular cross-sectional shape, which equally establishes the above-discussed desired relationships of the inhalation flow resistance to the exhalation flow resistance established by the second opening's shape.

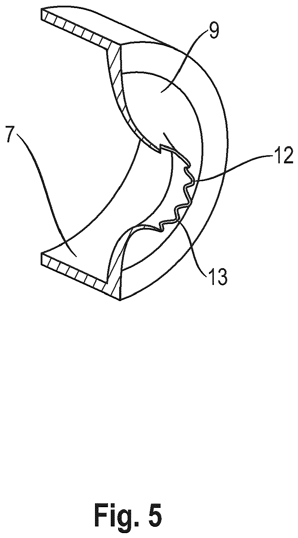

Accordingly, it may, as exemplarily illustrated in Fig. 5, also be possible to provide the end 12 with a circular cross-sectional shape. The desired inhalation flow resistance of 160Pa ± 30Pa inside the flow path 7 in the housing body 4, may, for example, be established upon inhalation of the user with a cross-sectional shape of the second opening 9 that continuously decreases along the second flow direction B and, thereby, results in an inner diameter of the second opening 9 of approximately 6.6mm, a radius of the illustrated bellmouth (can also be understood as a suction bellmouth) of 6.5mm and a radius of the "horn" of 5mm.

It is apparent from Fig. 5 that the end 12 of the second opening 9 may, irrespective of the cross-sectional shape of the end 12, be provided with chevrons 13 that positively affect the noise propagation due to inhalation (and exhalation) through the inhalation therapy device. These "saw-tooth"-patterns at the end 12 of the second opening 9, also known as chevrons 13, enable that any undesired acoustic side effects can be omitted during therapy with the inhalation therapy device.

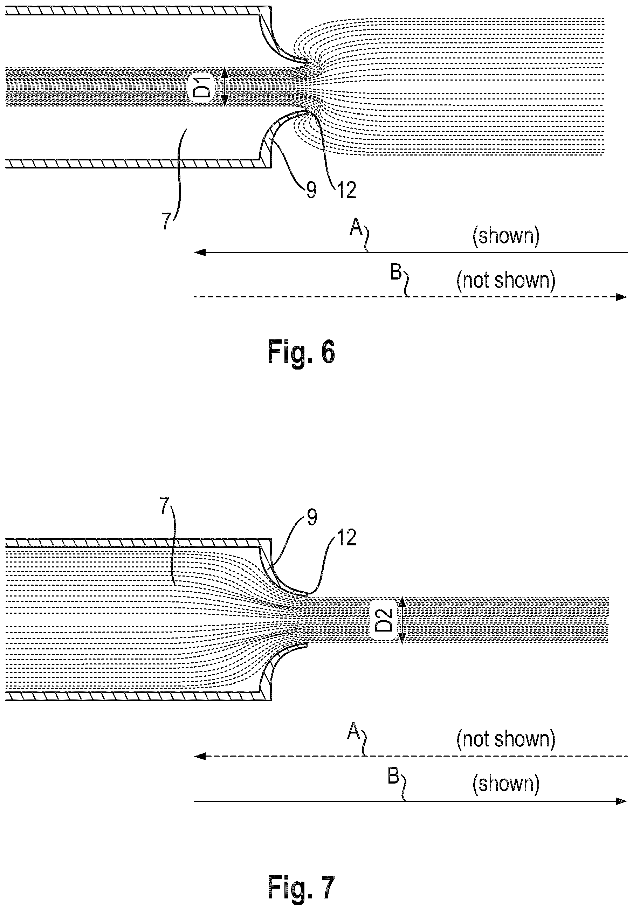

To facilitate the understanding of the second opening's shape allowing it to act as a flow restrictor, it will now be referenced to Figs. 6 and 7, which show an exemplary geometry and streamlines of an exemplary inhalation flow (Fig. 6) and an exemplary exhalation flow (Fig. 7) through the flow path's second opening 9. Since Fig. 6 illustrates the inhalation flow, the arrow for the first flow direction A is highlighted in bold, while the arrow for the (non-illustrated) second flow direction B is illustrated as a dashed line in Figure 6. Since Fig. 7 illustrates the exhalation flow, the arrow for the second flow direction B is highlighted in bold, while the arrow for the (non-illustrated) first flow direction A is illustrated as a dashed line.

It is apparent from Fig. 6 that air being sucked into the flow path 7 via the second opening 9 that is acting as a flow restrictor is guided towards a sharp edge, namely the end 12 of the second opening 9 that is oriented towards the outside of the housing body 4. Irrespective of its oval, polygonal, or, for example, circular cross-sectional shape, this fluid-dynamically sharp edge (i.e., of the end 12) to the exterior of the housing body 4 reduces the effective cross-section for the inhalation flow as a result of flow separations. It can be taken from Fig. 6 that air passes the end 12 of the second opening in the first flow direction A at a first flow diameter D1 only. This provokes comparably high velocities of the flow passing through the second opening 9 and, therefore, comparably high flow resistances. Accordingly, a flow resistance upon inhalation of the user is achieved via the shape of the second opening 9.

With reference to Fig. 7, it is apparent that these flow restricting effects are generally not given in the exhalation flow direction B. This is achieved because the second opening 9 is shaped in such a manner that in the second flow direction B the air flow is not confronted with any sharp edges, such that the full cross-section of the second opening 9 and particularly the end 12 of the second opening 9 to the exterior of the housing body 4 can be used by the air being expelled to the outside during exhalation of the user. This is illustrated via the second flow diameter D2 in Fig. 7. It is apparent that the first flow diameter D1 (the inhalation flow diameter), which is shown in Fig. 6, is smaller than the second flow diameter (the exhalation flow diameter) at the end 12 of the second opening 9 (D1<D2). Accordingly, velocities of the flow passing through the second opening 9 are comparably low and, therefore, flow resistances are comparably low. In summary, the fixed geometry passive valve is using the hysteresis effect between the two flow directions. Here, the flow resistance upon exhalation shall not exceed 135Pa, preferably 80Pa, measured at the peak flow of the sinus-shaped breathing pattern of two seconds of inhalation time and exhalation time, respectively, and 500ml of tidal volume, as those 135Pa do not lead to an unpleasant feeling during exhalation in combination with the increased inhalation resistance.

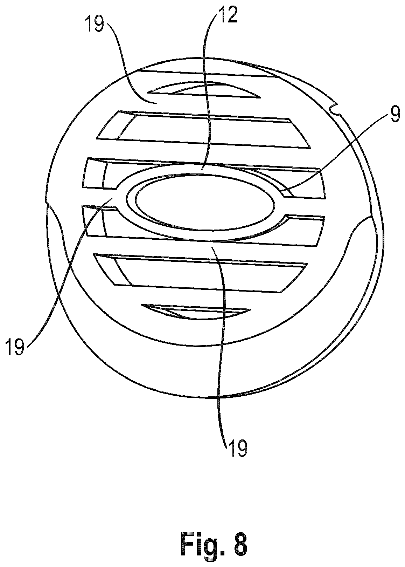

In addition to the various exemplary shapes of the second opening 9 illustrated in Figs. 3, 4, and 5, Fig. 8 shows another exemplary configuration of the second opening 9. In the embodiment illustrated in Fig. 8, which can be combined with any of the shapes of the second opening 9 illustrated in Figs. 3, 4, and 5, the end 12 of the second opening 9 is additionally provided with a protective member. In the exemplary embodiment of the protective member of Figure 8, the protective member is embodied via a plurality of ribs that surround the circumference of the end 12 of the second opening 9. This enables to protect the "sharp edge" of the second opening 9 being open to the environment of the inhalation therapy device 1 from any damages or mechanical stresses.

In a further preferred configuration, the second opening 9 may be arranged in such a manner in the housing body 4 that the end 12 of the second opening 9 is flush with an outer circumference of the housing body 4. This enables to protect the end 12 of the second opening 9 from any damages or mechanical stresses.

All of the aspects discussed with references to Figs. 2 to 8 are based on a scenario, in which the entire flow volume of the inhalation and the exhalation are guided through the second opening 9 of the flow path 7.

Yet, in another embodiment, it may also be possible that another, third opening 14 to the outside of the housing body 4 is provided in the flow path 7 between said first opening 8 and the second opening 9.

This is, for example, illustrated in Fig. 1. The third opening 14 is arranged upstream of the membrane unit 3 when seen in the first flow direction A. Further, the third opening 14 is provided with a check valve 15, which is configured to restrict a flow through the third opening 14 in the first flow direction A. That is, the check valve 15 prohibits any flow through the third opening 14 of the flow path 7 upon inhalation. However, the check valve 15 is also configured to enable a flow through the third opening 14 in the second flow direction B from the first opening 8 to the second opening 9 for enabling a partial expel of air through the third opening 14.

Irrespective of the presence of a third opening 14 or not, the inhalation therapy device 1 may, in a further, not illustrated embodiment, comprise an air filter device, which is reversibly attachable and detachable to the housing body 4. Said air filter device is configured to filter harmful substances from the aerosol. Provided that the third opening 14 is present, the air filter device is shaped to cover said third opening 14,its check valve 15, and the second opening 9, such that the flow in the second flow direction B passes through the air filter device to the outside of the housing body 4 upon exhalation. Vice versa, if the third opening 14 is not present and the flow path 7 extends between the first opening 8 and the second opening 9 only, the air filter device is shaped to cover the second opening 9, which is, besides the first opening 8, the only opening of the flow path 7 to the outside of the housing body 4, such that the entire flow volume along the first flow direction A passes through the air filter device upon inhalation and the entire flow volume along the second flow direction B passes through the air filter device upon exhalation.

According to an even further, non-illustrated embodiment, the end 12 of the second opening 9 that is oriented towards the outside of the housing body 4 may be provided with an elastic protective member. This elastic protective member aims to protect the above-mentioned sharp edge of the end 12 of the second opening 9 and is shaped in such a manner that the second opening remains open even upon inhalation and/or exhalation of the user through the inhalation therapy device 1.

### Reference Signs List

- 1: inhalation therapy device
- 2: reservoir
- 3: membrane unit
- 4: housing body
- 5: membrane
- 5.1: first side of the membrane
- 5.2: second side of the membrane
- 6: actuator
- 7: flow path
- 8: first opening
- 9: second opening
- 10: sensor
- 11: measurement port
- 12: end
- 13: chevrons
- 14: third opening
- 15: check valve
- 16: holding portion
- 17: aerosolization portion
- 18: latch
- 19: protective member (ribs)
- A: first flow direction
- B: second flow direction
- D1: first flow diameter
- D2: second flow diameter

## Claims

1. Inhalation therapy device (1), comprising:
a housing body (4),
a reservoir (2) for holding a liquid,
a membrane unit (3) comprising:
a membrane (5) disposed in the housing body (4) and having a plurality of apertures, wherein the membrane (5) is disposed so that, when liquid is held in the reservoir (2), the liquid is supplied to a first side (5.1) of the membrane (5), and
an actuator (6) coupled to the membrane (5) for vibrating the membrane (5), whereby the liquid passes through the apertures and an aerosol is generated at a second side (5.2) of the membrane (5) opposite to the first side (5.1) of the membrane (5),
wherein the inhalation therapy device (1) further comprises a flow path (7) being defined in the housing body (4) and having a first opening (8) to the outside of the housing body (4) at one end and a second opening (9) to the outside of the housing body (4) at another end, so that a flow is generatable in at least a first flow direction (A) from the second opening (9) to the first opening (8) in the flow path (7) upon inhalation of a user at the first opening (8) for entraining and delivering the generated aerosol, and in a second flow direction (B) from the first opening (8) to the second opening (9) in the flow path (7) upon exhalation of the user at the first opening (8) into the flow path (7); and
wherein the membrane unit (3) is disposed in the flow path (7) between the first opening (8) and the second opening (9),
**characterized in that**
the second opening (9) is shaped to act as a flow restrictor configured to establish a flow resistance upon inhalation of the user of 160Pa ± 100Pa, preferably 160Pa ± 60Pa, more preferably 160Pa ± 30Pa, and a flow resistance upon exhalation that is at least 25% lower than the flow resistance upon inhalation, measured at a peak flow of a sinus-shaped breathing pattern of two seconds of inhalation time and exhalation time, respectively, and 500ml of tidal volume.

2. Inhalation therapy device (1) according to claim 1, wherein the inhalation therapy device (1) further comprises a sensor (10) comprising a measurement port (11) being arranged in the flow path (7), preferably upstream of the membrane unit (3) when seen in the first flow direction (A), the sensor (10) being configured to detect a flow parameter of the flow in the flow path (7) via the measurement port (11).

3. Inhalation therapy device (1) according to any of the preceding claims, wherein the flow path (7) is shaped in such a manner that, upon inhalation, the entire flow volume along the first direction (A) to the first opening (8) passes through the second opening (9).

4. Inhalation therapy device (1) according to any of the preceding claims, wherein the second opening (9) is arranged upstream of the membrane unit (3) when seen in the first flow direction (A).

5. Inhalation therapy device (1) according to any of the preceding claims, wherein the second opening (9) is arranged at an upper half of the housing body (4), when the inhalation therapy device (1) is held by a user during therapy in a sitting position having an upright position of the user's head.

6. Inhalation therapy device (1) according to any of the preceding claims, wherein the cross-sectional shape of the second opening (9) gradually decreases when seen along the second flow direction (B) from the first opening (8) to the second opening (9).

7. Inhalation therapy device (1) according to any of the preceding claims, wherein an end (12) of the second opening (9) that is oriented towards the outside of the housing body (4) comprises an oval, a polygonal, preferably a rectangular, or a circular cross-sectional shape.

8. Inhalation therapy device (1) according to any of the preceding claims, wherein the end (12) of the second opening (9) that is oriented towards the outside of the housing body (4) comprises chevrons (13).

9. Inhalation therapy device (1) according to claims 1 to 7, wherein a protective member is arranged at the end (12) of the second opening (9) that is oriented towards the outside of the housing body (4), the protective member being shaped in such a manner that the second opening (9) remains open even upon inhalation and/or exhalation of the user through the inhalation therapy device (1),
wherein the protective member is a plurality of ribs, fins, or chevrons (13),
or
wherein the protective member is an elastic protective member and, preferably, the elastic protective member comprises chevrons (13).

10. Inhalation therapy device (1) according to any of the preceding claims, wherein the flow path (7) is shaped in such a manner that, upon inhalation, the entire flow volume along the first direction (A) passes through the second opening (9), and, upon exhalation, the entire flow volume along the second direction (B) passes through the second opening (9), and
wherein the second opening (9) is shaped as a fixed geometry passive valve configured to generate a flow resistance upon inhalation in the first flow direction (A) that is at least 25%, preferably 50%, more preferably 75%, most preferably 90% higher than upon exhalation in the second flow direction (B), measured at the peak flow of the sinus-shaped breathing pattern of two seconds of inhalation time and exhalation time, respectively, and 500ml of tidal volume.

11. Inhalation therapy device (1) according to claim 10, wherein the second opening (9) is formed by a plurality of sub-openings that are respectively shaped as a fixed geometry passive valve, and
wherein the sub-openings are configured to generate together a flow resistance upon inhalation in the first flow direction (A) that is at least 25%, preferably 50%, more preferably 75%, most preferably 90% higher than upon exhalation in the second flow direction (B), measured at the peak flow of the sinus-shaped breathing pattern of two seconds of inhalation time and exhalation time, respectively, and 500ml of tidal volume.

12. Inhalation therapy device (1) according to claim 10 or 11, wherein the second opening (9) is shaped in such a manner that the flow resistance upon inhalation in the first flow direction (A) is between 25% to 500%, preferably between 50% to 300%, more preferably between 75% to 200% higher than upon exhalation in the second flow direction (B).

13. Inhalation therapy device (1) according to any of claims 10 to 12, wherein the second opening (9) is shaped in such a manner that the flow resistance upon exhalation in the second flow direction (B) does not exceed 135Pa, preferably 100Pa, more preferably 80Pa, measured at the peak flow of the sinus-shaped breathing pattern of two seconds of inhalation time and exhalation time, respectively, and 500ml of tidal volume.

14. Inhalation therapy device (1) according to any of claims 10 to 13, wherein the inhalation therapy device (1) further comprises an air filter device, which, preferably, is reversibly detachable to the housing body (4),
wherein the air filter device is configured to filter harmful substances from the aerosol, and
wherein the air filter device (1) is shaped to cover the second opening (9) such that upon inhalation, the entire flow volume along the first flow direction (A) passes through the air filter device, and, upon exhalation, the entire flow volume along the second flow direction (B) passes through the air filter device.

15. Inhalation therapy device (1) according to any of claims 1 to 9, wherein a third opening (14) to the outside of the housing body (4) is provided in the flow path (7) between the first opening (8) and the second opening (9), and
wherein the third opening (14) is provided with a check valve (15), which is configured to restrict a flow through the third opening (14) in the first flow direction (A) and is configured to enable a flow through the third opening (14) in the second flow direction (B) from the first opening (8) to the second opening (9) for enabling at least a partial, preferably substantially a full, expel of air through the third opening (14).

16. Inhalation therapy device (1) according to claim 15, wherein the third opening (14) is provided upstream of the membrane unit (3) in the flow path (7) when seen along the first flow direction (A).

17. Inhalation therapy device (1) according claim 15 or 16, wherein the inhalation therapy device (1) further comprises the air filter device, which, preferably, is reversibly detachable to the housing body (4),
wherein the air filter device is configured to filter the aerosol, and
wherein the air filter device is shaped to cover the third opening (14), such that the flow in the second flow direction (B) passes through the air filter device to the outside of the housing body (4) upon exhalation.
